# EUROPEAN PATENT APPLICATION

(11) **EP 2 204 369 A1**
(43) Date of publication of application: **07.07.2010**
(21) Application number: 09180267.8
(22) Date of filing: 22.12.2009
(51) Int. Cl.: C07D 471/08, C07C 35/22, C07C 47/445

(54) **Process for preparing varenicline and intermediates for use therein**

(30) Priority: 22.12.2008 US 140025 P; 22.12.2008 US 140027 P; 22.12.2009 US 140031 P
(71) Applicant: Medichem, S.A., 08970 Barcelona (ES)
(72) Inventor: Szabó, András, Budapest H-1182 (HU); Papp, Attila Ákos, Budapest H-1048 (HU); Soto, José Javier, Alicante Apartado 99, E-03080 (ES); Winter, Stephen Benedict David, Barcelona E-08690 (ES)
(74) Representative: Gallafent, Alison

(57) **Abstract**

The present invention relates to an improved process for preparing varenicline, or pharmaceutically acceptable salts or solvates thereof, and in particular an improved process for preparing varenicline, or pharmaceutically acceptable salts or solvates thereof, employing intermediate compounds 1,2,3,4-tetrahydro-1,4-methano-naphthalene-*cis*-2,3-diol (a compound of Formula III), and / or indane-1,3-dicarbaldehyde (a compound of Formula IV) as prepared by a process of the present invention.

## Description

The present invention relates to an improved process for preparing varenicline, or pharmaceutically acceptable salts or solvates thereof, and in particular an improved process for preparing varenicline, or pharmaceutically acceptable salts or solvates thereof, employing intermediate compounds 1,2,3,4-tetrahydro-1,4-methano-naphthalene-*cis*-2,3-diol (a compound of Formula III), and / or indane-1,3-dicarbaldehyde (a compound of Formula IV).

Varenicline (a compound I of formula I) is the international commonly accepted non-proprietary name for 7,8,9,10-tetrahydro-6,10-methano-6*H*-pyrazino[2,3-*h*][3]benzazepine (which is also known as 5,8,14-triazatetracyclo[10.3.1.0^{2,11}.0^{4,9}]-hexadeca-2(11),3,5,7,9-pentaene), and has an empirical formula of C₁₃H₁₃N₃ and a molecular weight of 211.26.

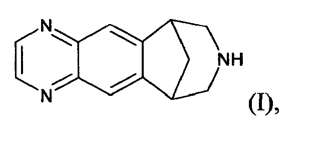

The L-tartrate salt of varenicline is known to be therapeutically useful and is commercially marketed for the treatment of smoking addiction. Varenicline L-tartrate is a partial agonist selective for α₄β₂ nicotinic acetylcholine receptor subtypes. In the United States, varenicline L-tartrate is marketed under the trade mark Chantix and is indicated as an aid to smoking cessation treatment.

Varenicline base and its pharmaceutically acceptable acid addition salts are described in U.S. Patent No. 6,410,550. In particular, the preparation of varenicline provided in this reference makes use of 10-aza-tricyclo[6.3.1.0^{2,7}]-dodeca-2(7),3,5-triene (a compound of Formula VI), as a key intermediate compound (see Scheme 1 below). Specifically, Example 1 of U.S. Patent No. 6,410,550 describes the synthetic preparation of key intermediate compound of Formula VI as depicted in Scheme 1.

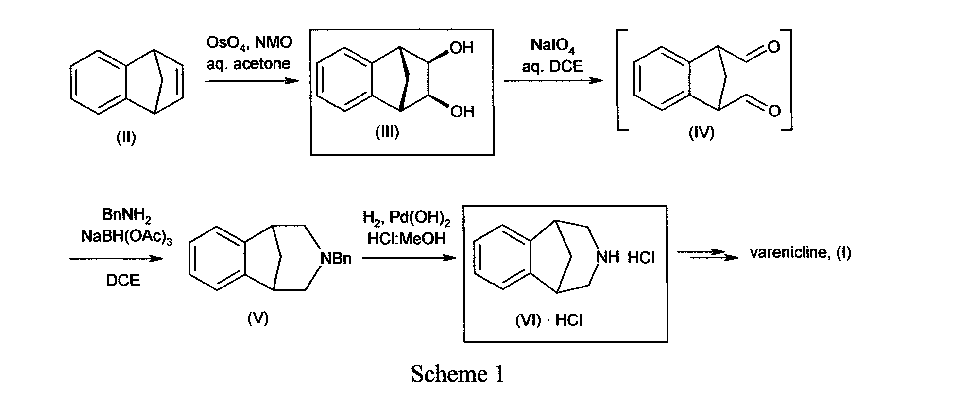

The process described in this reference uses 1,4-dihydro-1,4-methano-naphthalene (a compound of Formula II) as starting compound, which is converted into key compound of Formula VI via osmium-catalyzed dihydroxylation, followed by oxidative cleavage of intermediate compound (III), reductive amination of dialdehyde compound of Formula IV, and final debenzylation.

However, the *cis*-dihydroxylation step of a compound of formula II described in Scheme 1 is not suitable for preparing varenicline as it suffers from an important drawback, i.e. makes use of free osmium tetroxide as an oxidizing agent. Osmium tetroxide is a volatile and toxic substance (human tolerance: 0.002 mg/m³ of air), which requires particularly careful handling. Osmium tetroxide is an extremely dangerous and non desirable oxidizing agent, especially for industrial scale up, and its use entails large capital expenditures in plant safety equipment rendering the process not cost-effective. Further, this osmium-mediated hydroxylation process requires the final removal of the associated toxic osmium waste, which also entails safety concerns and as such is not desirable, especially for industrial implementation.

In view of the foregoing, there is, therefore, a need for providing new processes for preparing a compound of Formula III and / or a compound of formula IV, and as such new processes for preparing varenicline, or pharmaceutically acceptable salts or solvates thereof, which alleviate the above problems currently associated with known varenicline synthesis. In particular, there is a need for providing new processes for preparing a compound of Formula III and / or a compound of formula IV, and as such new processes for preparing varenicline, or pharmaceutically acceptable salts or solvates thereof, which avoid the use of free osmium tetroxide, and as such do not require the handling of associated toxic osmium waste from the process stream. Furthermore, such processes should be reliable and cost-effective, and as such useful for the industrial preparation of varenicline, or pharmaceutically acceptable salts or solvates thereof.

The present invention now provides improved processes for preparing 1,2,3,4-tetrahydro-1,4-methano-naphthalene-*cis*-2,3-diol (a compound of Formula III), and / or indane-1,3-dicarbaldehyde (a compound of Formula IV), and to the use thereof as intermediates for the synthesis of varenicline, or pharmaceutically acceptable salts or solvates thereof.

In particular, the present invention relates to an improved process for preparing a compound of Formula III, and / or a compound of Formula IV, from 1,4-dihydro-1,4-methano-naphthalene (a compound of Formula II), which process does not use free osmium tetroxide, does not require the handling of associated toxic osmium waste from the process stream, and which is reliable and cost-effective and which hence might be suitable for industrial implementation. In this regard, it has to be noted that the *cis* configuration of a compound of Formula III is a key factor to facilitate the preparation of a compound of Formula IV from a compound of Formula II, and as such for the synthesis of varenicline. While there are a number of potential methods available to *trans*-dihydroxylating a compound of Formula II, the *cis*-dihydroxylation of a compound of Formula II without free osmium tetroxide has been neither described nor suggested.

According to the present invention, therefore, there is provided a process for preparing varenicline, or pharmaceutically acceptable salts or solvates thereof, said process comprising an intermediate step of *cis*-dihydroxylating the alkene group of 1,4-dihydro-1,4-methano-naphthalene (a compound of formula II), 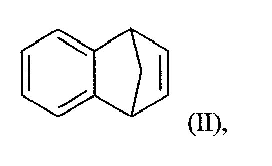 by treatment with an oxidizing agent selected from the group consisting of: (i) polymer supported osmium tetroxide, (ii) a permanganate salt, and (iii) ruthenium tetroxide, to obtain an intermediate product that is subsequently converted to varenicline, or pharmaceutically acceptable salts or solvates thereof.

In a first aspect, a process according to the present invention involves preparation of an intermediate compound which is 1,2,3,4-tetrahydro-1,4-methano-naphthalene-*cis-*2,3-diol (a compound of Formula III), and which can be subsequently converted to varenicline, or pharmaceutically acceptable salts or solvates thereof, 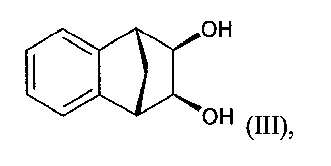 said process comprising:
(i) *cis*-dihydroxylating the alkene group of 1,4-dihydro-1,4-methano-naphthalene (a compound of formula II), 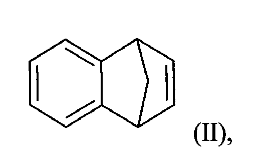 by means of treatment with polymer supported osmium tetroxide as an oxidizing agent, optionally in the presence of a suitable co-oxidizing agent, and in the presence of a suitable solvent comprising water, thereby obtaining a mixture comprising a compound of Formula III; and
(ii) optionally, isolating a compound of Formula III from the mixture.

Polymer supported osmium tetroxide as referred to herein for use in a process according to the first aspect of the present invention preferably comprises osmium tetroxide anchored onto a polymeric support and is suitably employed in the form of fibres. A particularly preferred polymer supported osmium tetroxide for use in a process according to the first aspect of the present invention is available under the trade mark FibreCat 3003. Polymer supported osmium tetroxide in the form of fibres, such as the above osmium product available under the trade mark FibreCat 3003, are oxidizing agents which in the present invention have been proved to be useful for the *cis*-dihydroxylation of a compound of formula II. Further, since polymer supported osmium tetroxide is in the form of fibres, use thereof combines the advantages of homogenous catalysts, i.e. reactivity and selectivity, with the advantages of heterogeneous catalysts, i.e. easy separation from the process stream by filtration. In addition, given that the osmium tetroxide is anchored onto a support, the osmium tetroxide is non-volatile and non-hazardous. Further, supporting the osmium in this manner also allows it to be easily removed from the reaction and allows its possible reuse.

The inventors have surprisingly observed that by using polymer supported osmium tetroxide, such as the above osmium product available under the trade mark FibreCat 3003, in a process according to the first aspect of the present invention, the reaction proceeds efficiently and does not show the drawbacks associated with the processes of the prior art, i.e. does not use toxic and volatile osmium tetroxide, and does not require the handling of associated toxic osmium waste from the process stream.

Suitably polymer supported osmium tetroxide as employed in a process according to the first aspect of the present invention is present either in catalytic amounts or in stoichiometric amounts. Preferably, the above osmium tetroxide is present in catalytic amounts.

It is further preferred that the suitable co-oxidizing agent of the step (i) of a process according to the first aspect of the present invention is selected from the group consisting of hydrogen peroxide, sodium hypochlorite, oxygen, *tert*-butylhydroperoxide, and *N-*methylmorpholine-*N*-oxide. More preferably, the suitable co-oxidizing agent of above step (i) is *N*-methylmorpholine-*N*-oxide. Typically, the co-oxidizing agent is present in stoichiometric amounts in step (i) of a process according to the first aspect of the present invention.

It is further preferred that the suitable solvent comprising water as employed in step (i) of a process according to the first aspect of the present invention comprises a mixture of at least one C₁-C₈ alcohol and water. More preferably, the suitable solvent comprising water is a mixture of *tert*-butanol and water.

In a particularly preferred process according to the first aspect of the present invention the *cis*-dihydroxylating of step (i) is carried out by means of treatment with catalytic amounts of polymer supported osmium tetroxide as a suitable oxidizing agent, in the presence of stoichiometric amounts of *N*-methylmorpholine-*N*-oxide as a suitable co-oxidizing agent, and in the presence of a *tert*-butanol / water 3:1 mixture as a suitable solvent.

In a second preferred aspect of the present invention, there is further provided a process of preparaing above intermediate compound of Formula III, which can be subsequently converted to varenicline, or pharmaceutically acceptable salts or solvates thereof, 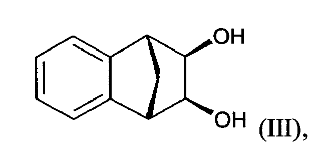 comprising
(i) *cis*-dihydroxylating the alkene group of 1,4-dihydro-1,4-methano-naphthalene (a compound of formula II), 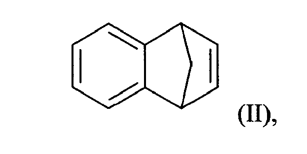 by means of (a) treatment with a suitable salt of permanganate as an oxidizing agent, optionally in the presence of a suitable co-oxidizing agent, and in the presence of a suitable solvent to obtain a first mixture, and (b) addition of a suitable hydrolising agent to the first mixture, thereby obtaining a second mixture comprising a compound of Formula III; and
(ii) optionally, isolating a compound of Formula III from the mixture.

The suitable salt of permanganate as an oxidizing agent of step (i)(a) of a process according to the second aspect of the present invention is preferably either an alkali metal salt of permanganate such as potassium permanganate salt or a quaternary ammonium salt of permanganate such as hexadecyl trimethyl ammonium permanganate salt. More preferably, the suitable salt of permanganate as an oxidizing agent of above step (i)(a) is hexadecyl trimethyl ammonium permanganate salt. The inventors have surprisingly observed that by using a suitable salt of permanganate in a process according to the second aspect of the present invention, the reaction proceeds efficiently and does not show the drawbacks associated with the processes of the prior art, *i*.*e*. does not use toxic osmium tetroxide, and does not require the handling of associated toxic osmium waste from the process stream.

It is further preferred that the permanganate salt as an oxidizing agent of the step (i)(a) of a process according to the second aspect of the present invention is present either in catalytic amounts or in stoichiometric amounts. Preferably, the above permanganate salt is present in stoichiometric amounts.

It is still further preferred that a solvent of the step (i)(a) of a process according to the second aspect of the present invention is preferably an ether solvent or mixtures thereof. More preferably, the above solvent is anhydrous tetrahydrofuran.

Typically, step (i)(a) of a process according to the second aspect of the present invention is carried out at a temperature in the range of about 20-30°C.

The hydrolising agent of the step (i)(b) of a process according to the second aspect of the present invention is preferably an aqueous basic solution. More preferably, the hydrolising agent is aqueous sodium hydroxide.

Typically step (i)(b) of a process according to the second aspect of the present invention is carried out at a temperature lower than about 10°C.

In a particularly preferred process according to the second aspect of the present invention the *cis*-dihydroxylating of the alkene group of a compound of Formula II of step (i) is carried out by means of (a) treatment with stoichiometric amounts of hexadecyl trimethyl ammonium permanganate salt as a suitable oxidizing agent, and in the presence of anhydrous tetrahydrofuran as a suitable anhydrous solvent at a temperature in the range of about 20-30°C to obtain a first mixture, and (b) addition of aqueous sodium hydroxide to the first mixture at a temperature lower than about 10°C.

In a third preferred aspect of the present invention, there is further provided a process of preparaing an intermediate compound which is indane-1,3-dicarbaldehyde (a compound of Formula IV), and which can be subsequently converted to varenicline, or pharmaceutically acceptable salts or solvates thereof, 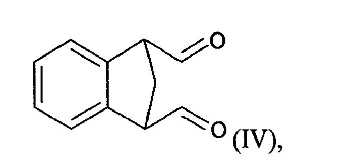 said process comprising:
(i) *cis*-dihydroxylating the alkene group of 1,4-dihydro-1,4-methano-naphthalene (a compound of formula II), 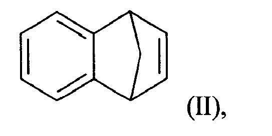 by means of treatment with ruthenium tetroxide as an oxidizing agent, optionally in the presence of a suitable co-oxidizing agent, and in the presence of a first solvent comprising water, thereby obtaining a first mixture comprising 1,2,3,4-tetrahydro-1,4-methano-naphthalene-*cis-*2,3-diol (a compound of Formula III), 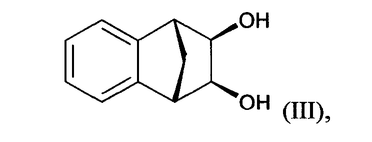
(ii) optionally, isolating a compound of Formula III from the first mixture;
(iii) oxidative cleaving of the *cis*-2,3-diol group of a compound of formula III by means of treatment with a suitable oxidizing agent, and in the presence of a second solvent comprising water, thereby obtaining a second mixture comprising a compound of Formula IV; and
(iv) optionally, isolating a compound of Formula IV from the second mixture.

The inventors have surprisingly observed that by using ruthenium tetroxide as an oxidizing agent in step (i) and a suitable oxidizing agent in step (ii) of a process according to the third aspect of the present invention, the preparation of a compound of formula IV proceeds efficiently and does not show the drawbacks associated with the processes of the prior art, i.e. does not use toxic osmium tetroxide, and does not require the handling of associated toxic osmium waste from the process stream.

Preferably, the ruthenium tetroxide as an oxidizing agent of step (i) of a process according to the third aspect of the present invention is generated *in situ* by means of oxidizing ruthenium (III) chloride with the co-oxidizing agent of step (i). Suitably, the above ruthenium tetroxide is present either in catalytic amounts or in stoichiometric amounts. Preferably, the ruthenium tetroxide is present in catalytic amounts.

Suitably the co-oxidizing agent of step (i) of a process according to the third aspect of the present invention is preferably a periodate salt. More preferably, the above co-oxidizing agent is sodium periodate salt.

Typically, the first solvent comprising water of step (i) of a process according to the third aspect of the present invention is a mixture of at least one alkylic nitrile and water. More preferably, the first solvent comprising water is a mixture of acetonitrile and water.

Suitably, the oxidizing agent of step (iii) of a process according to the third aspect of the present invention is a periodate salt. More preferably, the above oxidizing agent is sodium periodate salt.

Typically, the second solvent comprising water of step (iii) of a process according to the third aspect of the present invention is a mixture of at least one alkylic nitrile and water. More preferably, the second solvent comprising water is a mixture of acetonitrile and water.

It can be particularly preferred that a process according to the third aspect of the present invention is carried out via one-pot synthesis. Thus, the suitable co-oxidizing agent of step (i) is present in more than about 1.5 molar equivalents, preferably more than about 1.8 molar equivalents, so that it is also acting as the suitable oxidizing agent of step (iii). Additionally, the isolation of a compound of Formula III from the first mixture of step (ii) is not carried out so that the first solvent of the process of step (i) becomes the second solvent of the process of step (iii).

In a particularly preferred process according to the third aspect of the present invention the process comprises treating a compound of Formula II with catalytic amounts of ruthenium (III) chloride, in the presence of more than about 1.8 molar equivalents of sodium periodate salt, and in the presence of an acetonitrile / water 8:1 mixture, thereby obtaining a mixture comprising a compound of Formula IV.

Further aspects of the present invention comprise 1,2,3,4-tetrahydro-1,4-methanonaphthalene-*cis*-2,3-diol (a compound of Formula III), and / or indane-1,3-dicarbaldehyde (a compound of Formula IV) obtained by a process substantially as hereinbefore described and the use thereof for preparing varenicline, or pharmaceutically acceptable salts or solvates thereof.

In a further aspect, the present invention relates to a process for preparing varenicline (a compound of formula I), or pharmaceutically acceptable salts or solvates thereof, said process comprising:
(i) oxidative cleaving the *cis*-2,3-diol group of a compound of formula III as obtained by a process substantially as hereinbefore described, to obtain indane-1,3-dicarbaldehyde (a compound of Formula IV), 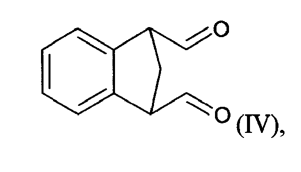
(ii) reductive aminating a compound of Formula IV, to obtain 3-benzyl-2,3,4,5-tetrahydro-1,5-methario-1*H*-3-benzazepine (a compound of Formula V), 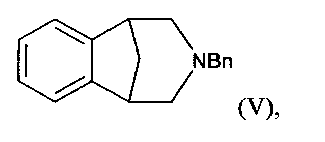
(iii) debenzylating a compound of formula V, to obtain 10-aza-tricyclo[6.3.1.0^{2.7}]-dodeca-2(7),3,5-triene (a compound of Formula VI), 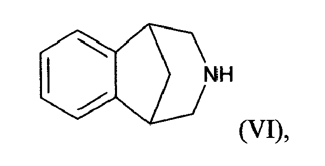 and;
(iv) transforming a compound of Formula VI into varenicline, or pharmaceutically acceptable salts or solvates thereof.

In a further aspect, the present invention relates to a process for preparing varenicline (a compound of formula I), or pharmaceutically acceptable salts or solvates thereof, said process comprising:
(i) reductive aminating a compound of Formula IV as obtained by a process substantially as hereinbefore described, to obtain 3-benzyl-2,3,4,5-tetrahydro-1,5-methano-1*H*-3-benzazepine (a compound of Formula V), 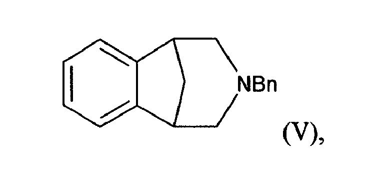
(ii) debenzylating a compound of formula V, to obtain 10-aza-tricyclo[6.3.1.0^{2,7}]-dodeca-2(7),3,5-triene (a compound of Formula VI), 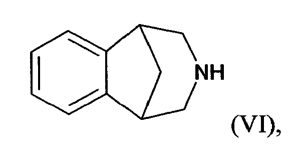 and;
(iii) transforming a compound of Formula VI into varenicline, or pharmaceutically acceptable salts or solvates thereof.

Suitably, reductive aminating of a compound of Formula IV of above step (i) comprises treating a compound of Formula IV with a suitable reducing agent and benzylamine, and in the presence of a suitable solvent.

The suitable reducing agent is preferably triacetoxyborohydride.

The suitable solvent is preferably 1,2-dichloroethane.

Varenicline as prepared according to the present invention is particularly suitable for use in the treatment of smoking addiction. There is further provided by the present invention, therefore, a pharmaceutically acceptable composition comprising an effective amount of varenicline, or pharmaceutically acceptable salts or solvates thereof substantially as hereinbefore described, together with a pharmaceutically acceptable carrier, diluent or excipient therefor. The term "effective amount" as used herein means an amount of varenicline which is capable of preventing, ameliorating or eliminating smoking addiction. By "pharmaceutically acceptable composition" is meant that the carrier, diluent or excipient must be compatible with varenicline and not be deleterious to a recipient thereof. Suitable pharmaceutically acceptable compositions according to the present invention are preferably for oral administration, and a particularly preferred oral dosage form comprises film coated tablets of varenicline.

The present invention further provides varenicline, or pharmaceutically acceptable salts or solvates thereof substantially as hereinbefore described, for use in the manufacture of a medicament for the treatment of smoking addiction and / or for the treatment of smoking addiction. The present invention also provides a method of treatment of smoking addiction, which method comprises administering to the patient an effective amount of varenicline substantially as hereinbefore described.

The present invention will now be further illustrated by reference to the following Examples, which do not limit the scope of the invention in any way.

### Specific examples

### General Experimental Conditions:

### GC Method A

The chromatographic separation was carried out in a HP-5 (cross-linked 5% phenyl methyl siloxanes), 15m length column with 0.53mm internal diameter and 1.5µm film thickness. The gas carrier was nitrogen. The column head pressure was 13psi. The injector temperature was 270°C and the detector temperature was 300°C. The temperature programme was as follows: Initial Temperature: 80°C; Initial time: 3 minutes; Rate: 15°C/min; Final temperature: 270°C; Final time: 30 minutes. The injection volume was 0.7 µL (manual). Test samples were prepared with a concentration of 4 mg/mL in ethyl acetate.

Approximate retention times and relative retention times of compounds II, III and IV are depicted in Table 1 below.

**Table 1**

| **Compound** | **Retention Time (minutes)** | **Relative Retention Time (RRT)** |
|---|---|---|
| Compound II | 3.8 | 0.5 |
| Compound III | 8.6 | 1.1 |
| Compound IV | 7.9 | 1.0 |

### GC Method B

The chromatographic separation was carried out in a RTX-50, 30m length column with 0.32mm internal diameter and 0.25µm film thickness. Gas carrier was helium. The column head pressure was 10psi. The injector temperature was 200°C and the detector temperature was 250°C. The temperature programme was as follows: Initial Temperature: 60°C (isotherm 2 minutes); Rate: 10°C/min until 100°C; Rate: 20°C/min until 250°C (isotherm 10 minutes). The injection volume was 0.1µL (manual). Test samples were prepared with a concentration 350-400 mM in methylene chloride.

Approximate retention times and relative retention times of compounds II and V are depicted in Table 2 below.

**Table 2**

| **Compound** | **Retention Time (minutes)** | **Relative Retention Time (RRT)** |
|---|---|---|
| Compound II | 9.2 | 0.6 |
| Compound V | 15.4 | 1.0 |

### GC Method C

The chromatographic separation was carried out in a VF-17ms, 30m length column with 0.32mm internal diameter and 0.25µm film thickness. Gas carrier was helium. The column head pressure was 10psi. The injector temperature was 250°C and the detector temperature was 250°C. The temperature programme was as follows: Initial Temperature: 100°C; Rate: 20°C/min; Final temperature: 250°C; Final time: 10 minutes. The injection volume was 0.1µL (manual). Test samples were prepared with a concentration 350-400mM in methylene chloride.

Approximate retention times and relative retention times of compounds II and V are depicted in Table 3 below.

**Table 3**

| **Compound** | **Retention Time (minutes)** | **Relative Retention Time (RRT)** |
|---|---|---|
| Compound II | 8.5 | 0.5 |
| Compound V | 15.6 | 1.0 |

### NMR analysis

NMR analyses were performed on a Varian Gemini 2000, 200 MHz, using DMSO-d6 as a solvent.

### Example 1: Preparation of 1,2,3,4-tetrahydro-1,4-methano-naphthalene-cis-2,3-diol (a compound of Formula III)

A 10mL round bottom flask was charged with a compound of formula II (142mg, 1mmol), *N*-methylmorpholine-*N*-oxide (120mg, 1.03mmol), *tert*-butanol (3mL) and water (1mL). FibreCat^{™} 3003 (OsO₄ anchored onto a polymeric support) (11.6mg, 0.0025mmol) was added to this solution and the mixture was heated to reflux. Complete conversion to a compound of formula III was detected by GC, method A, after 48h.

### Example 2: Preparation of 1,2,3,4-tetrahydro-1,4-methano-naphthalene-cis-2,3-diol (a compound of Formula III)

### Step A) Preparation of hexadecyl-trimethylammoniumpermanganate (HTAP):

HTAP was prepared from ion exchange reaction between hexadecyltrimethylammoniumbromide and potassium permanganate.

Potassium permanganate (17.38g, 0.11mol, 1equiv.) was dissolved in 500mL water. A solution of hexadecyltrimethylammoniumbromide (40.10g, 0.11mol, 1equiv) in 500mL water was added drop-wise over 45 min at 20-22°C, and the mixture stirred for 30 minutes at this temperature. The precipitated solid was collected by filtration, washed with water (3 x 100mL) and dried under vacuum at 35°C for 24 hours to give 34.38g of HTAP as a light purple solid.

### Step B) Preparation of a compound of formula III:

Compound II (3.52g, 24.8mmol, 1equiv.) was dissolved in anhydrous tetrahydrofuran (80mL) and a solution of HTAP (10g, 24.8mmol, 1.0equiv.) in anhydrous tetrahydrofuran (125mL) was added drop-wise at 23-30°C over 45min. The reaction was monitored by TLC (hexane-ethyl acetate = 1:1). After complete reaction the mixture was cooled to below 10°C, and methyl *tert*-butyl ether (50mL) and 5% aqueous NaOH solution (50mL) were added and the mixture stirred for 30min. The solid was removed by filtration, and washed with methyl *tert*-butyl ether (2 x 30mL). The combined layers of the filtrate were separated and the aqueous phase extracted with methyl *tert*-butyl ether (2 x 30mL). The organic layers were combined and washed with 5% aqueous NaOH solution (50mL), water (2 x 50mL), dried over MgSO₄, filtered and concentrated to obtain a dark green solid. This residue was suspended in acetone (15mL) and collected by filtration, washing with additional acetone (3 x 5mL). The product was dried under vacuum at 40°C to give 2.215g (50.7% yield) as a white crystalline solid.

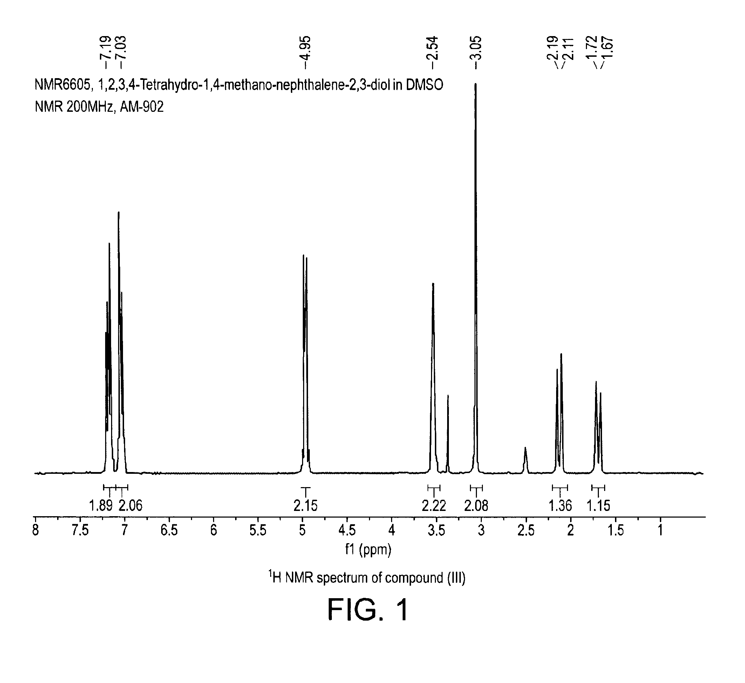

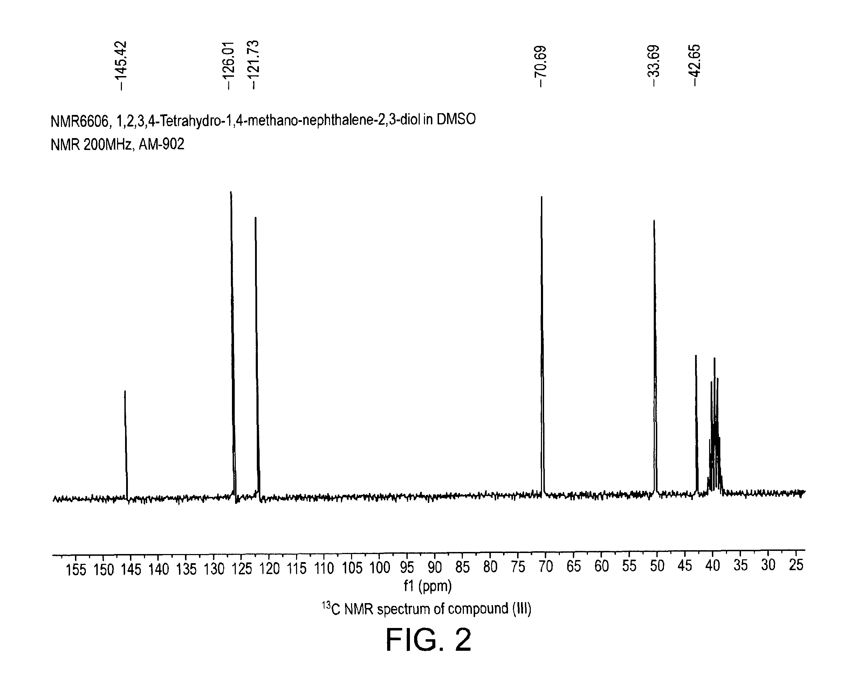

*Analytical data*: m.p. = 178.8-179.3°C; ¹H-NMR: See Figure 1; ¹³C-NMR: See Figure 2.

### Example 3: Preparation of indane-1,3-dicarbaldehyde (a compound of Formula IV)

A 25 mL round bottom flask was charged with a compound of formula I (142mg, 1mmol), Ruthenium (III) chloride hydrate (Aldrich, Reagent Plus^{™}) (7.2mg, 0.035mmol), acetonitrile (8.5mL) and water (1.1mL). The solution was heated to 45°C and sodium periodate (449mg, 2.1mmol) was added portionwise over 25 minutes. After 1h, the reaction was cooled to ambient temperature and filtered. The solids were washed with ethyl acetate (3 x 2mL) and water (3mL). The filtrate was concentrated under vacuum and 5mL of water were added to the obtained residue. The mixture was extracted with ethyl acetate (2 x 5mL) and the combination of the organic layers was washed with water (3 x 5mL), dried with MgSO₄ and concentrated under vacuum to obtain a compound of formula IV (118mg) in 68% yield, 70.9% purity (analyzed by GC, method A).

### Example 4: Preparation of 3-benzyl-2,3,4,5-tetrahydro-1,5-methano-1H-3-benzazepine (a compound of Formula V)

### Step A) Preparation of a compound of formula IV:

A 1000mL three necked round bottom flask equipped with magnetic stirrer, thermometer and reflux condenser, was charged with a compound of formula II (11.38g, 80mmol), Ruthenium (III) chloride hydrate (Aldrich, Reagent Plus^{™}) (581mg, 2.80mmol), acetonitrile (680mL) and water (85mL). The solution was heated to 43-47°C and sodium periodate (35.93g, 168mmol) was added in small portions over 25 minutes at 43-47°C. After 1h, the reaction was cooled to ambient temperature and filtered off. The filtrated solids were washed with 1,2-dichloroethane (2 x 50mL) and water (200mL). The filtrate was concentrated to 1/4 volumes under vacuum and the residual oil was extracted with 1,2-dichloroethane (4 x 75mL). After the, the combined organic phase was washed with water (4 x 100mL) and dried over MgSO₄ to get a solution of indane-1,3-dicarbaldehyde in 1,2-dichloroethane.

### Step B) Preparation of a compound of formula V:

A 1000mL three necked round bottom flask equipped with magnetic stirrer, thermometer and 500mL dropping funnel was charged with sodium triacetoxyborohydride (STAB, 27.23g, 128mmol) and 1,2-dichloroethane (190mL) and it was cooled at 0-5°C. In a 1000mL Erlenmeyer vessel were mixed benzylamine (9.17mL) and the previously obtained solution of indane-1,3-dicarbaldehyde in 1,2-dichloroethane, it was stirred for 2 minutes and immediately transferred to the dropping funnel. This mixture was added into the flask over 20 minutes at 0-5°C. After stirring for 1 hour at 20-25°C water (200mL) was added and the phases were separated. The aqueous phase was washed with 1,2-dichloroethane (2 x 80mL), the organic phases were combined, dried over MgSO₄ and concentrated to obtain 24.54g of a red brown oil. This crude product was distilled at 220°C at 1.8mbar to get 4.91g of a compound of formula V as a yellow oil (yield: 19.7%; purity: 80.7%, analyzed by GC, method B).

### Example 5: Preparation of 3-benzyl-2,3,4.5-tetrahydro-1.5-methano-1H-3-benzazepme (a compound of Formula V)

### Step A) Preparation of a compound of formula IV:

In a 2 L three necked vessel a compound of formula II (25g, 176mmol), acetonitrile (1500mL), water (188mL) and Ruthenium (III) chloride hydrate (Aldrich, Reagent Plus^{™}) (1.276g, 6.16mmol) were mixed and warmed up to 43-47°C. Then, sodium periodate (76.69g, 331mmol) was added in small portions over 30 minutes at 43-47°C. The reaction mixture was stirred at 43-47°C for 30 minutes, cooled down to 20-25°C and filtered off. The filtrate was washed with 1,2-dichloroethane (2 x 75mL). To the mother liquor water (400mL) was added and concentrated to 1/3. The precipitated solids were filtered and to the mother liquor 1,2-dichloroethane (270mL) was added. The phases were separated and the aqueous phase was extracted with 1,2-dichloroethane (2 x 120mL). The combined 1,2-dichloroethane phases were washed with water (4 x 120mL), dried over MgSO₄ and concentrated to about 400mL. The resulting solution of indane-1,3-dicarbaldehyde was dried again over MgSO₄.

### Step B) Preparation of a compound of formula V:

A suspension of sodium triacetoxyborohydride (STAB, 59.61g, 280mmol) in 1,2-dichloroethane (475mL) was cooled down to 0-5°C. To this suspension, a mixture of the previously obtained solution of indane-1,3-dicarbaldehyde in 1,2-dichloroethane and benzylamine (20.16mL) was dropped at 0-5°C over 35 minutes and then allowed to warm up until 20-25°C. After stirring for 1 hour at 20-25°C the reaction mixture was quenched with saturated aqueous solution of sodium carbonate (200mL) and then stirred for 1 more hour at 20-25°C. The phases were separated and the aqueous phase was washed with dichloromethane (3 x 100mL). The combined organic phases were dried over MgSO₄ and concentrated to obtain 47.21 g of a red brown glass. This crude product was vacuum distillated at 116-122°C at 0.26- 0.32mbar to get 4.03g of a compound of formula V as a yellow oil (yield: 9.19%; purity: 86.0%, analyzed by GC, method C).

## Claims

1. A process for preparing varenicline, or pharmaceutically acceptable salts or solvates thereof, said process comprising an intermediate step of *cis-*dihydroxylating the alkene group of 1,4-dihydro-1,4-methano-naphthalene (a compound of formula II), 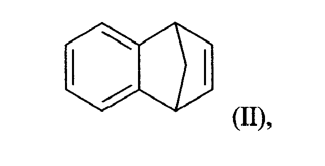 by treatment with an oxidizing agent selected from the group consisting of: (i) polymer supported osmium tetroxide, (ii) a permanganate salt, and (iii) ruthenium tetroxide, to obtain an intermediate product that is subsequently converted to varenicline, or pharmaceutically acceptable salts or solvates thereof.

2. A process for preparing 1,2,3,4-tetrahydro-1,4-methano-naphthalene-*cis*-2,3-diol (a compound of Formula III), 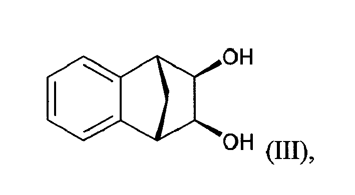 said process comprising:
(i) *cis*-dihydroxylating the alkene group of 1,4-dihydro-1,4-methano-naphthalene (a compound of formula II), 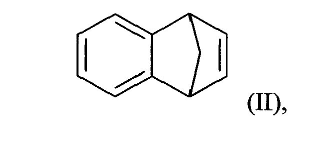 by means of treatment with polymer supported osmium tetroxide as an oxidizing agent, optionally in the presence of a suitable co-oxidizing agent, and in the presence of a suitable solvent comprising water, thereby obtaining a mixture comprising a compound of Formula III; and
(ii) optionally, isolating a compound of Formula III from the mixture.

3. A process according to claim 2, wherein said polymer supported osmium tetroxide is: (a) in the form of fibres; and / or (b) is removed from the mixture by filtration; and / or (c) is present either in catalytic amounts, or stoichiometric amounts.

4. A process according to claim 2 or 3, wherein said co-oxidizing agent is at least one of the group consisting of hydrogen peroxide, sodium hypochlorite, oxygen, *tert-*butylhydroperoxide, and *N*-methylmorpholine-*N*-oxide, preferably *N-*methylmorpholine-*N*-oxide; and / or said solvent comprising water is a mixture of at least one C₁-C₈ alcohol with water, preferably a mixture of *tert*-butanol and water.

5. A process according to claim 2, wherein *cis*-dihydroxylating the alkene group of a compound of Formula II is carried out by means of treatment with catalytic amounts of polymer supported osmium tetroxide as an oxidizing agent, in the presence of stoichiometric amounts of *N*-methylmorpholine-*N*-oxide as a co-oxidizing agent, and in the presence of a *tert*-butanol / water 3:1 mixture as a solvent comprising water.

6. A process for preparing 1,2,3,4-tetrahydro-1,4-methano-naphthalene-*cis*-2,3-diol (a compound of Formula III), 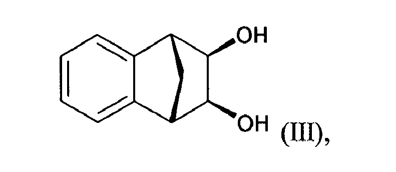 said process comprising:
*(i) cis*-dihydroxylating the alkene group of 1,4-dihydro-1,4-methano-naphthalene (a compound of formula II), 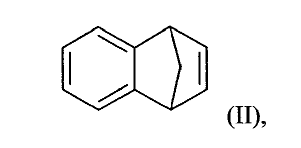 by means of (a) treatment with a salt of permanganate as an oxidizing agent, optionally in the presence of a co-oxidizing agent, and in the presence of a solvent to obtain a first mixture, and (b) addition of a hydrolising agent to the first mixture, thereby obtaining a second mixture comprising a compound of Formula III; and
(ii) optionally, isolating a compound of Formula III from the mixture.

7. A process according to claim 6, wherein said salt of permanganate as an oxidizing agent of step (i)(a) is either an alkali metal salt of permanganate or a quaternary ammonium salt of permanganate, preferably potassium permanganate salt or hexadecyl trimethyl ammonium permanganate salt, most preferably hexadecyl trimethyl ammonium permanganate salt, and wherein suitably said permanganate oxidizing agent is present in stoichiometric amounts.

8. A process according to claim 6 or 7, wherein said solvent of step (i)(a) is an ether solvent or mixtures thereof, preferably anhydrous tetrahydrofuran, and suitably step (i)(a) is carried out at a temperature in the range of 20-30°C, and / or said hydrolising agent of step (i)(b) is an aqueous basic solution, preferably aqueous sodium hydroxide, and wherein suitably step (i)(b) is carried out at a temperature lower than about 10°C.

9. A process according to any of claims 6 to 8, wherein *cis*-dihydroxylating of the alkene group of a compound of Formula II of step (i) is carried out by means of (a) treatment with stoichiometric amounts of hexadecyl trimethyl ammonium permanganate salt as a suitable oxidizing agent, and in the presence of anhydrous tetrahydrofuran as a suitable solvent at between about 20-30°C to obtain a first mixture, and (b) addition of aqueous sodium hydroxide to the first mixture at a temperature lower than about 10°C.

10. A process for preparing, optionally via one-pot synthesis, indane-1,3-dicarbaldehyde (a compound of Formula IV), 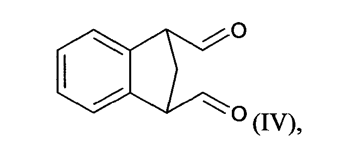 said process comprising:
*(i) cis*-dihydroxylating the alkene group of 1,4-dihydro-1,4-methano-naphthalene (a compound of formula II), 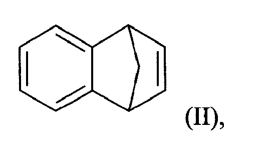 by means of treatment with ruthenium tetroxide as an oxidizing agent, optionally in the presence of a suitable co-oxidizing agent such as a periodate salt, especially sodium periodate, and in the presence of a first solvent comprising water, thereby obtaining a first mixture comprising 1,2,3,4-tetrahydro-1,4-methano-naphthalene-*cis*-2,3-diol (a compound of Formula III), 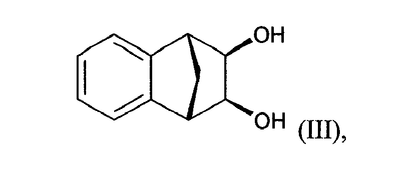
(ii) optionally, isolating a compound of Formula III from the first mixture;
(iii) oxidative cleaving of the *cis*-2,3-diol group of a compound of formula III by means of treatment with a suitable oxidizing agent, such as a periodate salt, especially sodium periodate, and where appropriate in the presence of a second solvent comprising water, thereby obtaining where appropriate a second mixture comprising a compound of Formula IV; and
(iv) optionally, isolating a compound of Formula IV from the second mixture.

11. A process according to claim 10, wherein the ruthenium tetroxide as an oxidizing agent of step (i) is preferably present in catalytic amounts and is generated *in situ* by means of oxidizing ruthenium (III) chloride with the co-oxidizing agent of step (i), and preferably one or both of said first or second solvent is a mixture of at least one alkylic nitrile and water, preferably a mixture of acetonitrile and water.

12. A process according to claim 10 or 11, which comprises treating a compound of Formula II with catalytic amounts of ruthenium (III) chloride, in the presence of more than about 1.8 molar equivalents of sodium periodate salt, and in the presence of an acetonitrile / water 8:1 mixture, thereby obtaining a mixture comprising a compound of Formula IV.

13. 1,2,3,4-tetrahydro-1,4-methano-naphthalene-*cis*-2,3-diol (a compound of Formula III) obtained by a process according to any of claims 2 to 9, and / or indane-1,3-dicarbaldehyde (a compound of Formula IV) obtained by a process according to any of claims 10 to 12, and preferably use thereof for preparing varenicline, or pharmaceutically acceptable salts or solvates thereof.

14. A process for preparing varenicline (a compound of formula I), 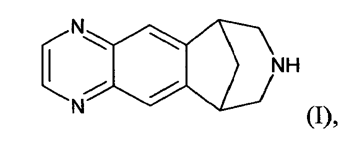 or pharmaceutically acceptable salts or solvates thereof, said process comprising:
oxidative cleaving the *cis*-2,3-diol group of a compound of formula III obtained according to any of claims 2 to 9, to obtain indane-1,3-dicarbaldehyde (a compound of Formula IV), 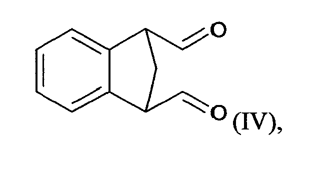
reductive aminating a compound of Formula IV, to obtain 3-benzyl-2,3,4,5-tetrahydro-1,5-methano-1*H*-3-benzazepine (a compound of Formula V), 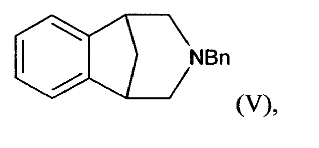
debenzylating a compound of formula V, to obtain 10-aza-tncyclo[6.3.1.0^{2,7}]-dodeca-2(7),3,5-triene (a compound of Formula VI), 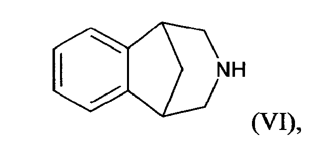
and;
transforming a compound of Formula VI into varenicline, or pharmaceutically acceptable salts or solvates thereof.

15. A process for preparing varenicline (a compound of formula I), 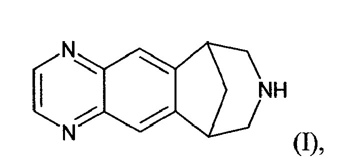 or pharmaceutically acceptable salts or solvates thereof, said process comprising:
(i) reductive aminating a compound of Formula IV obtained according to any of claims 10 to 12, preferably by treating a compound of Formula IV with a reducing agent such as triacetoxyborohydride and benzylamine, and suitably in the presence of a solvent such as 1,2-dichloroethane to obtain 3-benzyl-2,3,4,5-tetrahydro-1,5-methano-1*H*-3-benzazepine (a compound of Formula V), 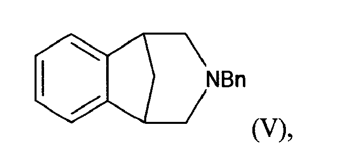
(ii) debenzylating a compound of formula V, to obtain 10-aza-tricyclo[6.3.1.0^{2,7}]-dodeca-2(7),3,5-triene (a compound of Formula VI), 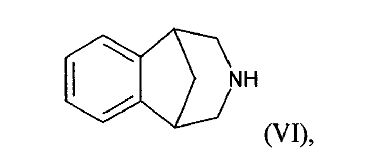 and;
(iii) transforming a compound of Formula VI into varenicline, or pharmaceutically acceptable salts or solvates thereof.
